# EUROPEAN PATENT SPECIFICATION

(11) **EP 4 293 124 B1**
(45) Date of publication and mention of the grant of the patent: **19.02.2025**
(21) Application number: 23185680.8
(22) Date of filing: 07.05.2020
(51) Int. Cl.: C12Q 1/6825

(54) **COMBINED SOLUTION PHASE AND SOLID PHASE DNA AMPLIFICATION**
KOMBINIERTE LÖSUNGSPHASE UND FESTPHASEN-DNA-AMPLIFIKATION
AMPLIFICATION D'ADN EN PHASE SOLIDE ET EN PHASE SOLUBLE COMBINÉE

(30) Priority: 08.05.2019 GB 201906461
(43) Date of publication of application: 20.12.2023
(62) Divisional of application: 20725896.3
(73) Proprietor: DNAe Diagnostics Limited, London W12 7RZ (GB)
(72) Inventor: DAVIDSON, David Allan, London (GB); WORSLEY, Graham, London (GB); KILLPACK, Jarrett, London (GB); REED, Samuel, Carlsbad (US)
(74) Representative: Stratagem IPM Limited

(56) References cited:
- WO-A2-2007/086935
- WO-A2-2015/162301
- JUNSUN KIM ET AL: "Multiplex real-time PCR using temperature sensitive primer-supplying hydrogel particles and its application for malaria species identification", PLOS ONE, vol. 13, no. 1, 2 January 2018 (2018-01-02), pages e0190451, XP055652762, DOI: 10.1371/journal.pone.0190451

## Description

### FIELD OF THE INVENTION

The present invention relates to a method for the sequence specific amplification of subpopulations of nucleic acid fragments from a broader population of nucleic acid sequences. The invention cleanly detects only the desired target sequences. Described is a means for the efficient targeted amplification and detection of a particular nucleic acid sequence from a population.

### BACKGROUND TO THE INVENTION

The identification of pathogens in blood and other biological samples is important for the effective treatment of numerous disease states including sepsis. Sepsis kills 5 people ever hour in the United Kingdom and 25% of all sepsis survivors suffer permanent, life-changing after effects. The early detection and identification of the causative agents of bacterial infections is essential to preventing the onset of, and successfully treating, sepsis. Current methods of detection and identification of blood-borne infection include blood culture and the use of antibiotic susceptibility assays. These methods involve the culturing of cells, which is costly, both in time and money. Often, septic shock will occur before cell culture results can be obtained.

The use of current molecular detection methods, such as PCR, which can identify the pathogen from a sample by analysing the nucleic acid from the pathogen, is limited by a low overall sensitivity level. This is especially a problem when analysing human samples due to the large amounts of non-target (human) nucleic acids present. These non-target nucleic acids can inhibit downstream purification and amplification of target (pathogen) nucleic acids leading to false negative results, or give false positive readings for pathogen nucleic acids due to non-specific amplification.

For example, there are several antibiotic resistance genes which have multiple variants due to point mutations or other polymorphisms, and where the presence of a particular mutation may result in a different therapeutic strategy compared to another mutation. It is important, therefore, that identification of these polymorphisms is done in a timely and cost-effective manner. Current identification strategies using PCR and other amplification reactions are limited in capability and laboratories can resort to sequencing to elucidate the internal sequence and identify the polymorphisms - this is time-consuming and costly to implement.

Selective amplification of a population of target nucleic acids relative to non-target nucleic acids is therefore of great value to the medical industry and to research.

Amplification reactions generally rely on a pair of amplification primers for each desired sequence to be amplified. Whilst a few pairs of non-immobilised primers can be combined, the combination of large numbers of pairs of primers is not generally feasible due to cross-hybridisations between primers. Thus multiplex PCR is generally done using separate primers in separate solution volumes, requiring a large amount of sample. The current inventions describe improvements to amplification reactions enabling sequence selective amplification of a plurality of different primers within a single sample volume.

Methods have also been disclosed for emulsion PCR, where individual templates are diluted such that water bubbles in an oil emulsion contain on average less than one template molecule per water bubble. Beads can be included having a single immobilised primer, the second primer being in solution. Thus amplification is performed using a mix of two primers, one of which is immobilised and one of which is in solution. This however only gives a single amplification product per reaction volume, in contrast to the methods described herein where the first amplification product is further amplified.

WO2007/086935 discloses an apparatus and method for performing rapid DNA sequencing such as genomic sequencing. The method includes the steps of preparing a sample DNA for genomic sequencing, amplifying the prepared DNA in a representative manner and performing multiple sequencing reaction on the amplified DNA with only one primer hybridisation step.

Amplification methods are also known where both primers are immobilised, such as for example Illumina's cluster based bridging amplification. In such cases there are no primers in free solution. Junsen Kim et al "Multiplex real-time PCT using temperature sensitive primer-supplying hydrogel particles and its application for malaria species identification" includes a brief overview of known methods, describing real-time PCR as a gold standard for analysing the genes of various diseases.

### SUM MARY

The current invention provides a biphasic amplification reaction comprising both solid and solution-phase (thermocycled or isothermal) amplification reactions that can use a combination of immobilised and non-immobilised primers and target DNA templates to simultaneously generate DNA amplicons in solution and on a surface. The presence absence of the amplicons can be detected in an end-point assay after amplification or in real time. This biphasic approach differs from the state of the art, including emulsion PCR, because it includes both solid, i.e. surface-based amplification and solution phase amplification.

A nucleic acid sample can be amplified using one or more non-immobilised primers. This solution-phase-generated DNA, as well as the target DNA template, can also act, or subsequently acts, as a template for an additional reaction on the solid phase whereby one or more immobilised primers interact with the solution-phase template as part of the DNA amplification reaction. By immobilising primers of different sequence in known locations and using a detection system capable of discriminating the location (for example, using ISFETs on CMOS IC chips or array based fluorescence/optical imaging systems) the readout provides the system with further discriminating powers.

Here, we propose a novel method to achieve multiplexed sequence specific amplification of subpopulations of nucleic acid fragments from a broader population of nucleic acid sequences.

A method is described that includes a method for the amplification and analysis of nucleic acid sequences comprising:
a. taking a system having a liquid volume in contact with a solid support wherein the liquid volume contains a nucleic acid sample, two or more non-immobilised amplification primers and reagents for nucleic acid amplification, and the solid support has one or more immobilised amplification primers;
b. performing a nucleic acid amplification reaction using the non-immobilised amplification primers to produce a first non-immobilised nucleic acid amplification product;
c. further amplifying the first non-immobilised nucleic acid amplification product using the one or more immobilised amplification primers to produce an immobilised second nucleic acid amplification product;
d. either
   i. interrogating a localised signal generated at the immobilised second nucleic acid amplification product location in real time or
   ii. removing the liquid volume and first non-immobilised amplification product and replacing with a new liquid volume having a primer which hybridises to the immobilised second nucleic acid amplification product, and either;
      1. directly detecting the hybridised primer or
      2. flowing over the immobilised second amplification product a solution comprising at least one species of nucleotide in order to extend the hybridised primer, and
e. detecting the presence, absence or sequence of the second nucleic acid amplification product.

The method can be performed wherein the non-immobilised amplification primers in the liquid volume are released from the solid phase prior to amplification. The nucleic acid amplification reaction using non-immobilised primers can use one or more pairs of non-immobilised primers. The nucleic acid amplification, of any stage, can be isothermal, or it can be carried out by thermocycling.

According to the method, the primer hybridised to the second nucleic acid amplification product can be fluorescently labelled and subsequent measurement of fluorescence can be achieved. Alternatively, the method can be performed wherein the detection of the amplified products uses detection of the released protons from nucleotide incorporation via, for example, an ISFET sensor.

The method can be performed in such a manner that the first and second amplification products are obtained using amplification primers having the same nucleic acid sequence. The method may further involve the immobilised amplification primers amplifying an internal section of the first amplification product such that the second amplification product is shorter than the first amplification product.

The method may further involve the exposure of the same first liquid volume to two or more immobilised amplification primers where each primer amplifies a different sequence. The two or more immobilised amplification primers may differ by a single base, thereby detecting single base variants in the first nucleic amplification process.

The immobilised primers of the method can be on an open microarray or in separate wells. The separate wells can be simultaneously exposed to the same reagent volume. If the wells contain immobilised primers of different sequence, then different amplicons can be generated in different wells, thus enabling multiplexed amplification within a single liquid volume.

The amplification products can be optically or electrically detected. To be detected optically, the amplification products need to be labelled with a fluorescent marker and the marker can then be detected through an optical regimen.

Whether optical or electrical output is detected, the sensing can be performed via an array of silicon based sensors, such as Field Effect Transistors (FETs). The FETs chosen may be any suitable CMOS chip including, but not limited to ISFETs. The sensors are configured to integrate signal sensed either temporally, or spatially or both in order to identify the presence or absence, or in some cases the quantum of amplification product present. Unlike some deployments of optical arrays, they are not configured to image the output.

### BRIEF DESCRIPTION OF THE DRAWINGS

Figure 1: Schematic showing Single Localised Anchored Primer Amplification and Detection (LAPAD)
Figure 2: Schematic showing Multiple Localised Anchored Primer Amplification and Detection (LAPAD)

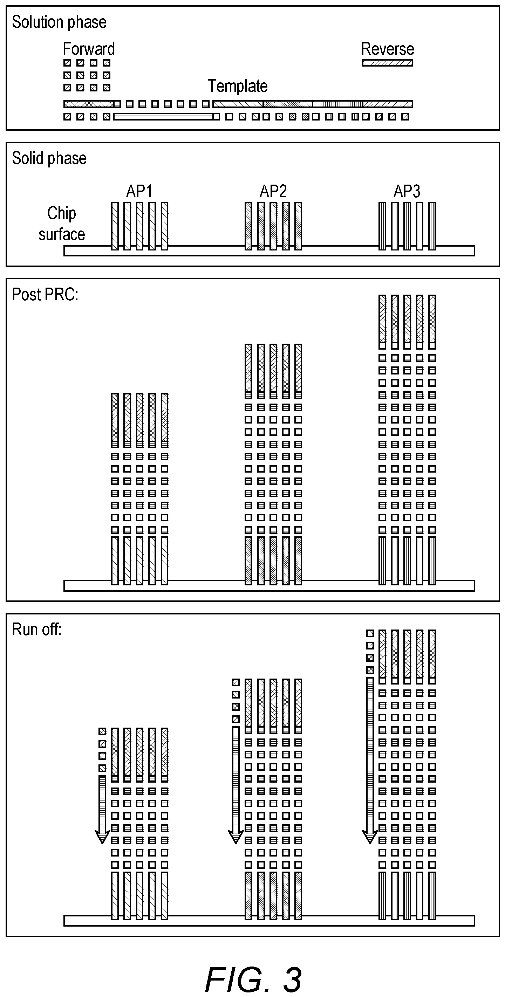

Figure 3: Schematic showing a run-off implementation. Takes place on an open system requiring flow capabilities on the chip. During the amplification reaction, product amplicon from the solution phase lands on the solid phase primers, which extend to create a forest of extended amplicon on the chip surface. Post amplification, the amplification product and reagents are washed away to leave single stranded amplicon on the chip surface. Run off primer and enzyme is loaded onto the chip, and, after a short hybridisation step, all four nucleotides are flowed in together, creating a burst of protons on those ISFETs with extended oligos. This proton burst is very clear to see with minimal data processing.

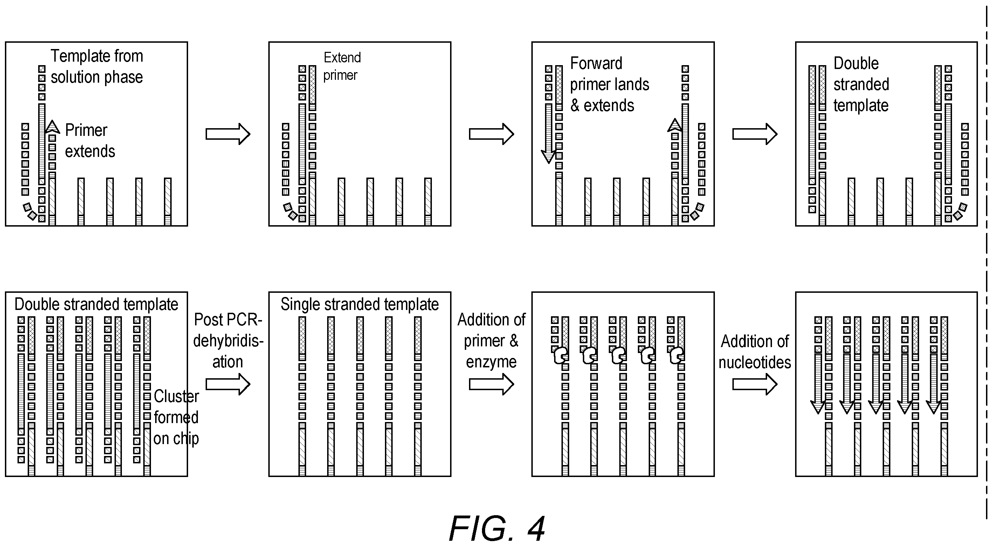

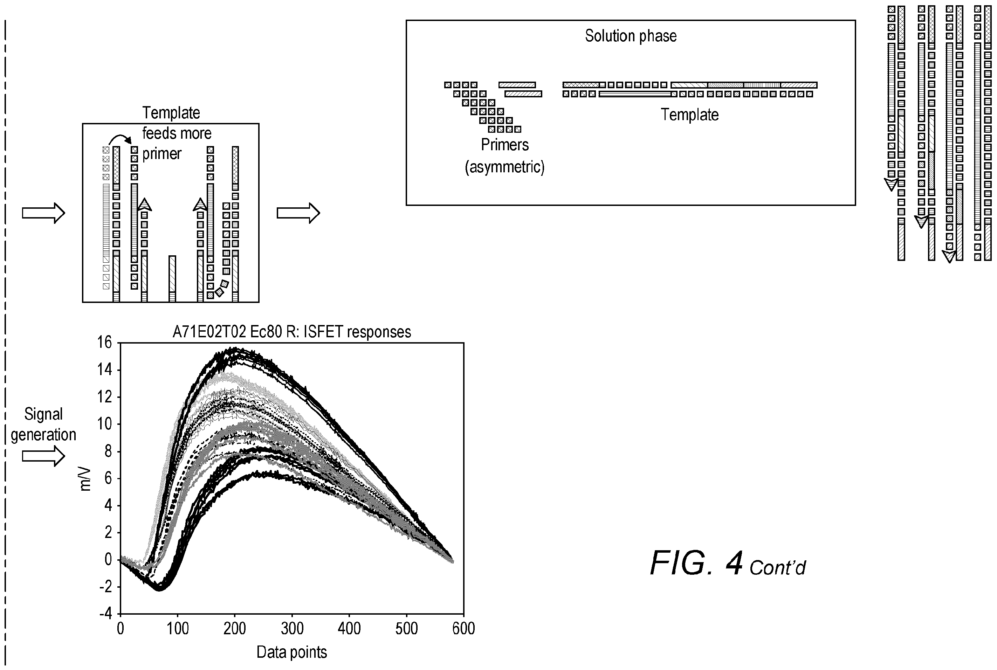

Figure 4 shows a run-off workflow and data therefrom. The first row represents the amplification stage of the assay, generating a cluster of extended amplicon on the chip surface. This is followed by a dehybridisation step, using heat for example, to produce single stranded amplicon on the chip surface with no product in solution phase. Following hybridisation of the run off primer and enzyme, all four nucleotides are flowed across the chip and a signal is generated on those ISFETs which have been specifically extended.

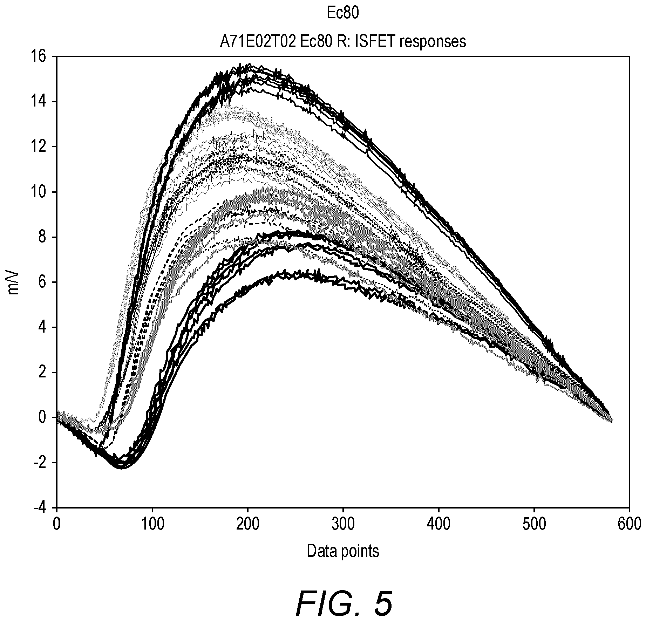

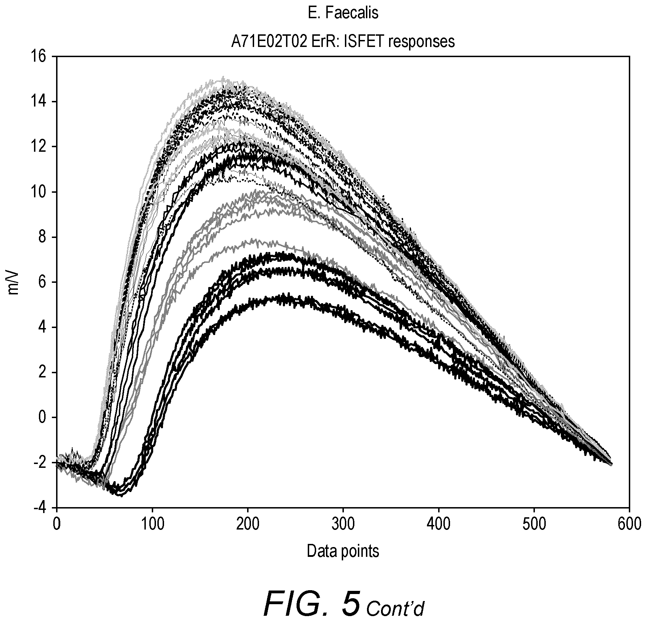

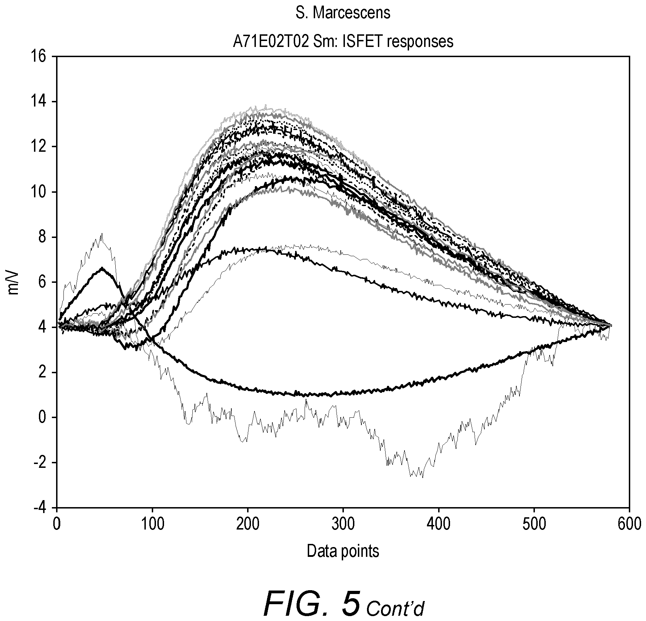

Figure 5 shows sample ISFET data showing run off signal generated following PCR for three target templates. Data shown is for E.coli (an 80 base product, therefore max 60 base run off), E. faecalis and S. marcescens following a specific amplification of the relevant target. Data is unmodified, with all ISFETs shown.

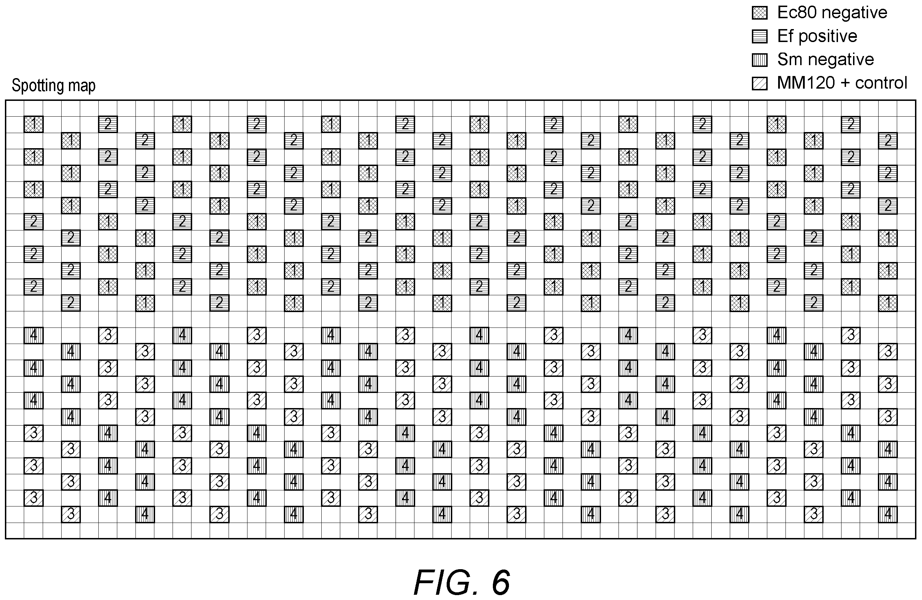

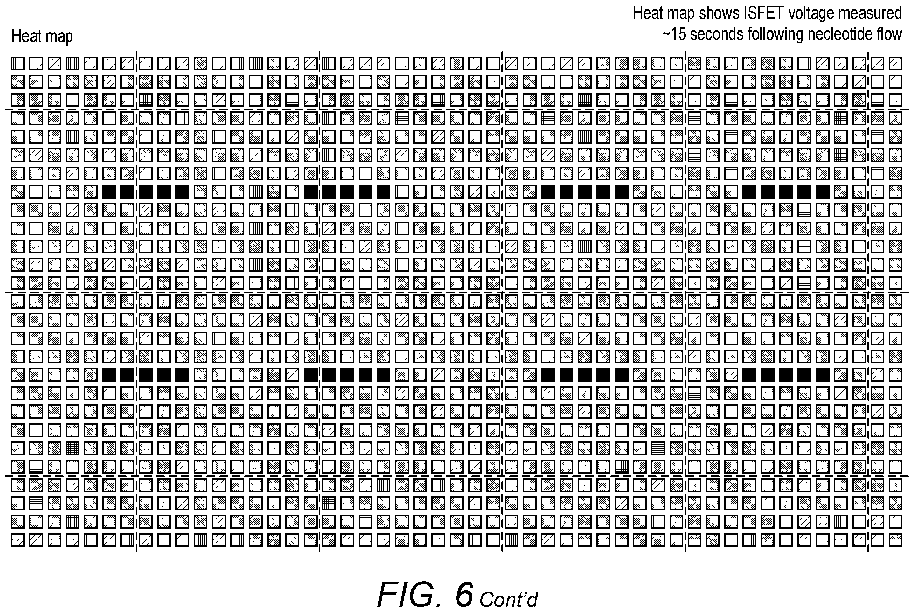

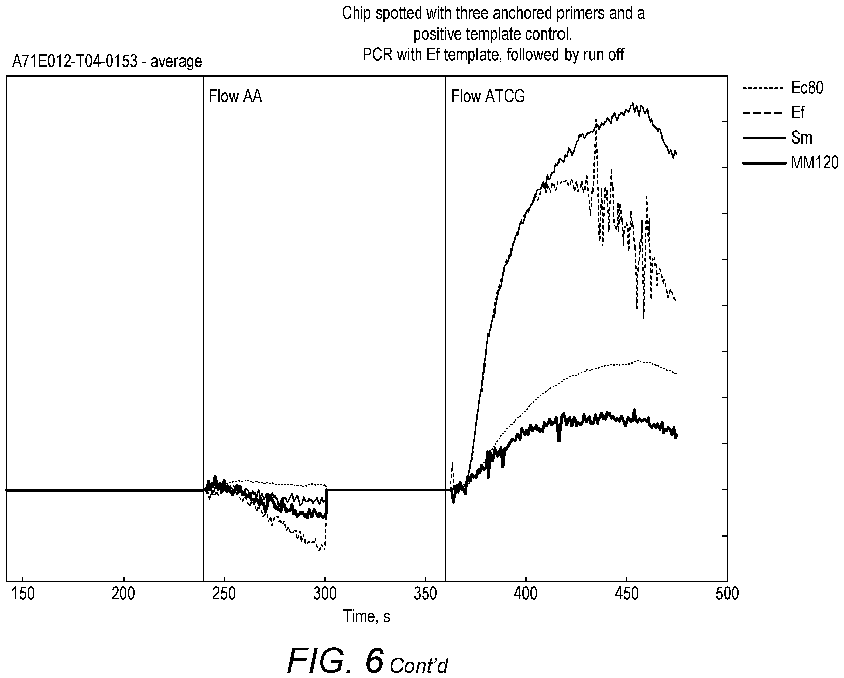

Figure 6 shows a multiplexed chip spotted with three anchored primers and a positive template control. PCR with Ef template, followed by Run Off. Heat map shows ISFET voltage measured -15 seconds following nucleotide flow. ISFETs can be seen matching the spotting pattern and correspond to both Ef and positive control. Trace on the right shows examples of ISFET responses for each of the ISFET groups.

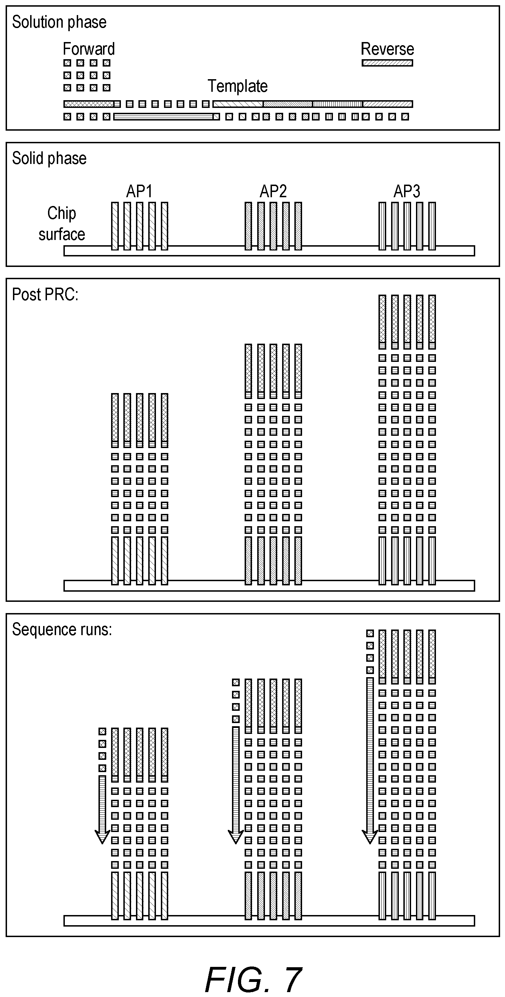

Figure 7 shows a sequencing implementation. Rather than all four nucleotides add for the run-off, nucleotides are added sequentially. Takes place on an open system requiring flow capabilities on the chip. During the amplification reaction, product amplicon from the solution phase lands on the solid phase primers, which extend to create a forest of extended amplicon on the chip surface. Post amplification, the amplification product and reagents are washed away to leave single stranded amplicon on the chip surface. Sequencing primer and enzyme is loaded onto the chip, and, after a short hybridisation step, all four nucleotides are flowed in individually, creating a burst of protons on those ISFETs with amplification product and the correct template base. Single base resolution can be achieved.

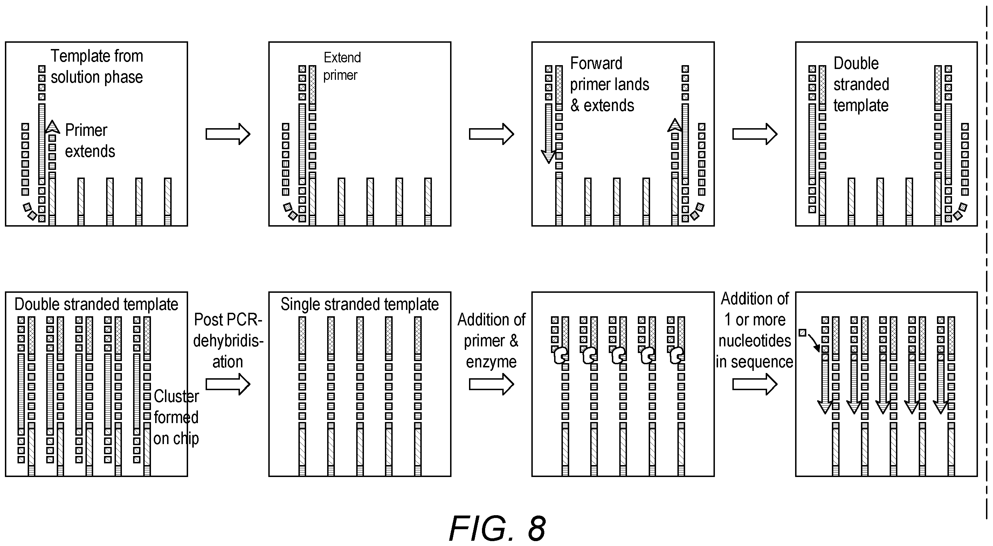

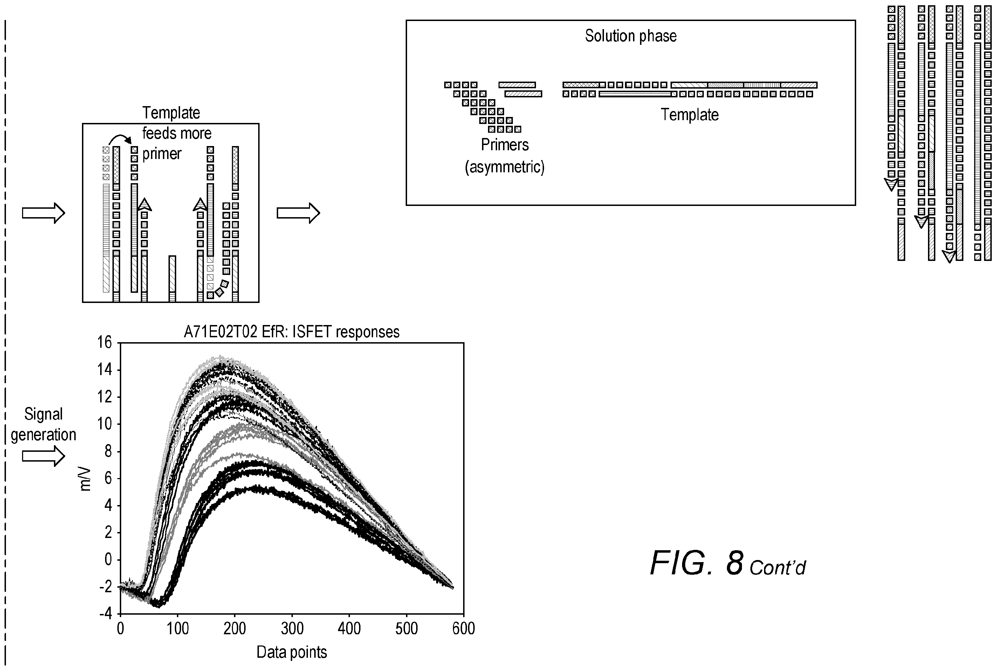

Figure 8 shows a sequencing workflow and data therefrom. The first row represents the amplification stage of the assay, generating a cluster of extended amplicon on the chip surface. This is followed by a dehybridisation step, using heat for example, to produce single stranded amplicon on the chip surface with no product in solution phase. Following hybridisation of the run off primer and enzyme, all four nucleotides are flowed individually across the chip and a signal is generated on those ISFETs where nucleotides were incorporated and corresponding to where amplification product was generated.

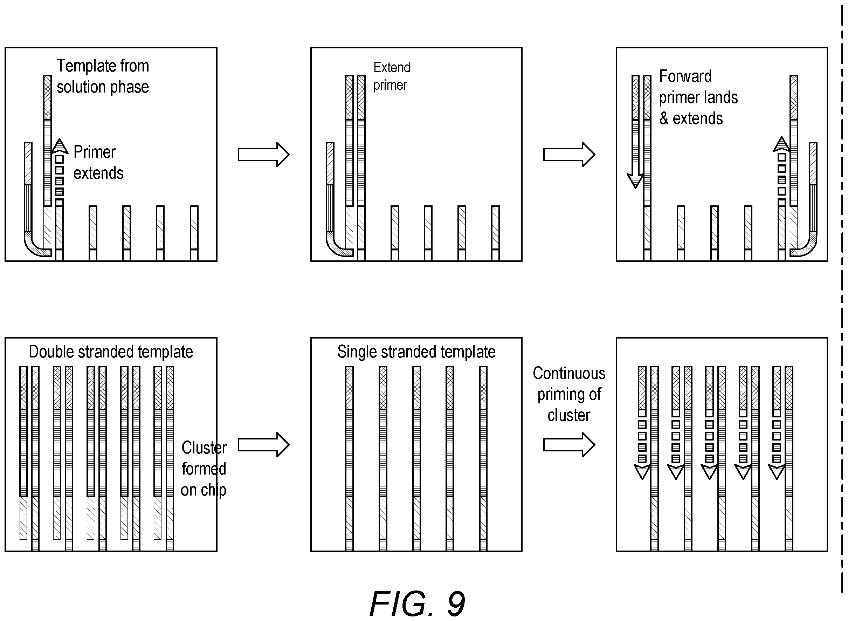

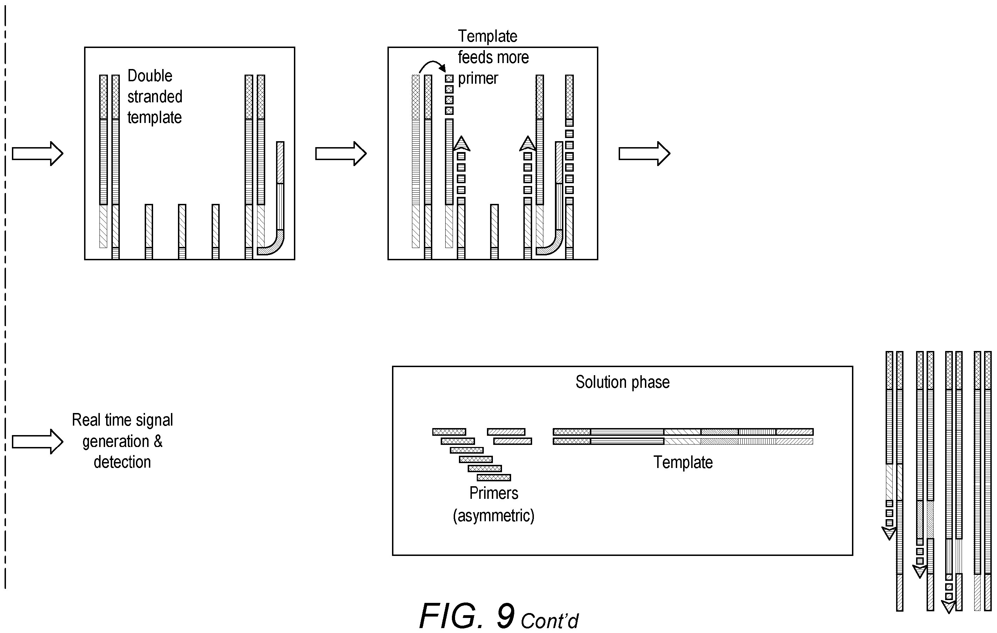

Figure 9 shows a real-time detection workflow. Can take place on a platform with no fluid flow post chip amplification, or with minimal fluid flow. The reaction could take place in a permanently-sealed reaction chamber or chambers. During the amplification reaction, product amplicon from the solution phase lands on the solid phase oligos, modifying and extending the specific oligos. In a sealed system with low buffering, protons will be generated close to solid phase during the extension stage of each cycle of the amplification reaction. Pushing the asymmetry to give a significant excess of forward primer in the solution phase will effectively generate a "mini Run off" burst of protons in each cycle which should become detectable in the later cycles. An alternative to an asymmetry in the primers at the start of the amplification reaction, additional primer could be released or added during the amplification reaction, or at set time points. The amplification reaction will be in low buffer to allow real time detection of the pH signal.

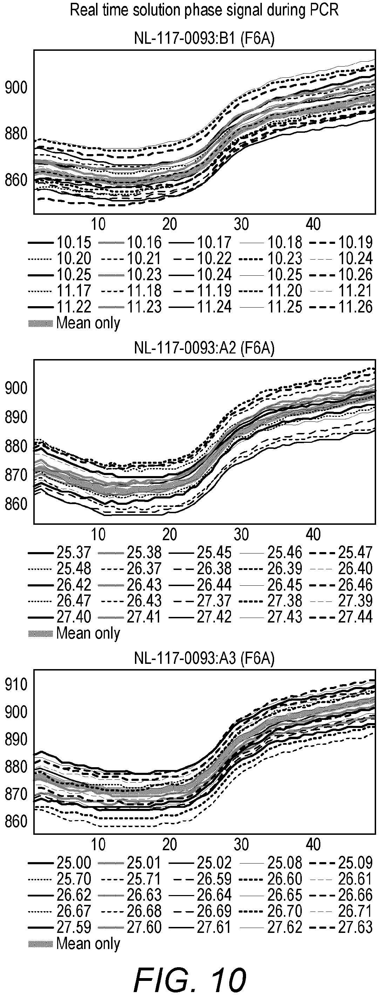

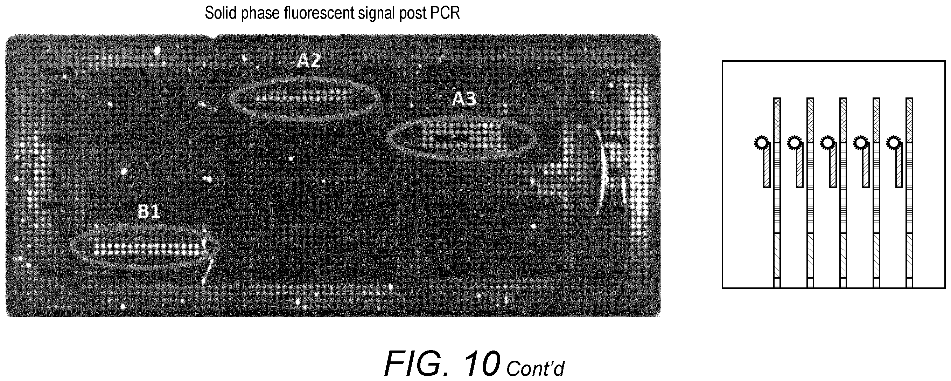

Figure 10 shows data from real-time detection. Chip spotted with multiple anchored primers in blocks, PCR (3:1 asymmetry) with 6 chamber gasket (3 chambers fluidically sealed), Run off in flow station with single chamber. Solution phase shows real time pH signal indicating that the reaction was progressing. As confirmation of amplification, post PCR, the chip was probed with a fluorescent label targeted against the distal end of the amplicon, and indicates that the anchored primers were extended during the PCR reaction.
Figure 11 shows average delta signal from ISFETs for Example 1.
Figure 12 shows delta signals from all ISFETS for Anchored Primer target Positive for Example 1.
Figure 13 shows Delta signals from all ISFETs for Anchored Primer target Negative for Example 1.
Figure 14 shows Delta signals from all ISFETs for Anchored Primer Control for Example 1.
Figure 15 shows average ISFET signal results for Positive, Negative and Control primers for Example 1.
Figure 16 is a gel electrophoresis image showing solution phase amplicon was generated post PCR for *Serratia marcescens,* for Example 1.
Figure 17 shows a Sensospot fluorescent image for Example 1.
Figure 18 shows average Delta signals from ISFETs for Example 2.
Figure 19 shows Delta signals for Anchored Primers targets 1 to 4 Positive for Example 2.
Figure 20 shows Delta signals from all ISFETs for Anchored Primer Negative for Example 2.
Figure 21 shows Delta signals from all ISFETs for Anchored Primer Control for Example 2.
Figure 22 shows average ISFET signal results for Positives, Negatives and Controls for Example 2.
Figure 23 is a gel electrophoresis image for Example 2.
Figure 24 is a Sensospot fluorescent image for Example 2.

### DETAILED DESCRIPTION OF THE INVENTION

A system is described with the capability to generate a readout of different sequences from the same reaction volume.

Briefly, the system comprises a biphasic amplification reaction wherein a solution phase (PCR or isothermal) amplification reaction occurs in the presence of immobilised primers on the solid phase which further participate in producing amplification reaction product. The solution-phase (PCR or isothermal) amplification reaction can use one or more primers and target nucleic acid template to generate nucleic acid amplification product in solution with a corresponding signal being detected by the system when sufficient product has been produced. This solution-phase-generated nucleic acid amplification product, as well as the target nucleic acid template, can also act, or subsequently acts, as a template for an additional reaction on the solid phase whereby one or more immobilised primers interact with the solution-phase template as part of the amplification reaction and generate a nucleic acid sequence which is subsequently detectable by the system. By immobilising the primers in known locations and using a detection system capable of discriminating the location (for example, using ISFETs on CMOS IC chips or array type optical imaging systems) the readout enables the selective amplification and detection of many amplicons in the same device.

An amplification reaction is carried out in the solution phase in the presence of one or more further primers immobilised on the solid phase. The immobilised primers can be either the same sequence as one or more of the solution phase primers or correspond to internal sequences of the nucleic acid template generated in the solution phase. The former allows a second call to be made from the same reaction components, and the latter a second call that adds confirmation and increased confidence that the correct amplification reaction product has been generated and/or gives further information on the internal sequence of the target template.

The detection mode described herein can use ISFET CMOS technology but would also be applicable to existing nucleic acid amplification reactions and microarray methods, as well as a customised system to integrate the real time detection of both solution and solid phase reactions. The redundancy potential of the ISFET CMOS allows a significant number of replicates for each assay, as well as multiple target genes for each target, thereby improving the confidence in the system by relying upon multiple readouts for each target. Incorporation of positive and negative controls in each reaction vessel is also facilitated by this design, ensuring that correct calls are made when the control perform as required.

Below are various embodiments of the combined solution and solid phase amplification reaction.

### DNA Amplification Readouts

The immobilised primers described can be the same as, or similar to, one or more of the solution phase primers, and participate in the amplification reaction such that an increased signal is generated from the immobilised primers when compared to an amplification carried out in the absence of the solution primers, thereby adding confidence in the result but without adding further information to the sequence being generated. For example, an asymmetric PCR in the solution phase can be compensated for by having the depleted primer in the solution phase present on the solid phase. As the amplification reaction progresses the solution phase product being generated is encouraged to interact with the solid phase, generating both solution phase and solid phase amplicons. The immobilised amplicon can be detected. Presence of this readout across multiple locations where the primer is immobilised gives increased confidence the then amplified material is definitely present in the sample. The confidence in the result is increased if immobilised primers having a different sequence do not give a positive result.

### DNA Amplification with Internal Confirmation

The immobilised primers described can correspond to internal sequences of the DNA template generated in the solution phase such that any signal generated on the solid phase is positive confirmation that the correct product has been made in the solution phase. This confirmation demonstrates that the solution phase amplicon has not been generated by such things as primer dimers, mismatched primers or other non-specific reaction (for example, signals generated by interacting with the host (human) DNA when the intended target is a pathogen in the host sample), and gives a significant benefit over standard confirmatory tests such as melt curve analysis and electrophoresis gel imaging in that additional sequence information is inferred in the readout.

The internal confirmation signal is analogous to standard laboratory confirmatory procedures such as melt curve analysis, electrophoresis gel imaging, etc.

Using immobilised primers corresponding to internal sequences in the target template allows further specificity to be given to the system whereby the combination of solution phase reaction and solid phase reaction identifies more sequence information than a solution phase reaction on its own, thereby improving the confidence in the result compared to solution phase alone.

### DNA Amplification with Internal Sequence Information

In addition to the confirmatory step already identified, the potential is also available to allow multiple immobilised primers to be present corresponding to different areas of the solution phase-amplified product, such that one or more can be used to provide internal information in addition to merely confirming that the correct product has been made in the solution phase.

This can take a simple form of multiple internal primers from the amplified product, each adding confidence in the inferred result by providing more than one sequence alignment, or in a more advanced version can be used to identify internal variations within a target gene corresponding to point mutations and other polymorphisms. For example, there are several antibiotic resistance genes which have multiple variants due to point mutations or other polymorphisms, and where the presence of a particular mutation may result in a different therapeutic strategy compared to another mutation. It is important, therefore, that identification of these polymorphisms is done in a timely and cost-effective manner. Current identification strategies using PCR and other amplification reactions are limited in capability and laboratories can resort to sequencing to elucidate the internal sequence and identify the polymorphisms - this is time-consuming and costly to implement and is often not done.

In this embodiment, a single primer pair can be used in the solution phase to amplify up all variants of the gene, with one or more immobilised primers corresponding to the point mutations or polymorphisms can be used on the solid phase to identify one or more of the variants. A combination of each of the internal immobilised primers provides the user with rich information on the overall sequence of the amplified gene and can result in clinical decisions being made easier and more quickly.

### Run-off (including multiple run-offs)

Amplification reactions are generally performed in high ionic strength buffers. For sequencing systems which measure the proton release of incorporated nucleotides, the amplification products can be detected if the amplification buffer is replaced by a buffer with a low buffering capacity. Alternatively the amplification products can be detected using fluorescently labelled primers or via the use of fluorescently labelled nucleotides.

The run-off takes place on an open system requiring flow capabilities on the chip. During the amplification reaction, product amplicon from the solution phase lands on the solid phase primers, which extend to create a forest of extended amplicon on the chip surface. Post amplification, the amplification product and reagents are washed away to leave single stranded amplicon on the chip surface. Run off primer and enzyme is loaded onto the chip, and, after a short hybridisation step, all four nucleotides are flowed in together, creating a burst of protons on those ISFETs with extended oligos. This proton burst is very clear to see with minimal data processing.

In order to perform an electronic/proton detection, at the end of the amplification stage described previously with both solution phase and solid phase reactions, the solution phase components are flushed out of the cartridge and replaced with a solution with low buffering capacity. This is then followed by primers and enzyme, either in sequence or combined, followed by a flow of nucleotides, which will result in a release of protons as the nucleotides are incorporated and a corresponding signal on the detection platform, for example a mV change on the relevant ISFETs. The primers used for the run-off can correspond to any location within the amplified template, including generic sequences added during earlier amplification reactions. Using amplicon specific primers internal to the solution amplified product delivers increased confidence in the result by confirming that the correct material has been made on the solid phase, but can add to the complexity of the system by having a higher multiplex. If generic sequences have been added in earlier amplification reactions, it would be possible to limit the scale of the solution phase multiplex thereby improving the efficiency of the reaction, however this may limit the readout confidence due to the potential for misfiring of the generic sequences.

By knowing the location of a particular immobilised primer, the identification of the target can be determined when the protons being generated during the run-off reaction result in a signal on the particular ISFET sensor to which the specific primer is immobilised. Multiple replicates for each assay target is possible in this embodiment, with the scale of the multiplex limited by spatial separation, the number of wells and sensors per device and bioinformatics challenges.

The system can make use of a single run-off reaction or multiple run-off reactions, with or without a dehybridisation step between each run. Additional run-offs can be used to interrogate the earlier signals such that increased confidence of the internal sequence can be made. For example, if generic sequences were used in earlier amplification reactions, a first run-off could use the generic sequence to identify which assays have generated signals, with subsequent run-off using specific primers corresponding to those particular assays to confirm that the correct product was made.

Multiple target sequences can be analysed in parallel as primers of different sequence can be immobilised in different locations on the solid support.

### Sequence information (single or multiple bases at a time)

At the end of the amplification stage described previously, the solution phase components can be flushed out of the cartridge and replaced with a solution with low buffering, as with the run-off workflow. This is then followed by primers and enzyme, either in sequence or combined, followed by a flow of individual nucleotides or combination of 2 or more nucleotides, which will result in a release of protons when the correct complementary nucleotides are flowed across the chip and incorporated. This workflow provides the richest information of all of the embodiments, in that it gives sequence information as opposed to relying only on the alignment of primers with their corresponding template. This workflow not only confirms that the correct alignment has taken place and the correct product has been made on the solid phase, but also indicates the internal sequence either by inference or by direct identification. Similar approaches can be used here as have been discussed in the run-off embodiment, with multiple runs being possible to provide further information, as well as a workflow that combines run-off with this approach of one or more nucleotides being used.

### Real-time detection

The amplification process can be detected in real time by measuring the extension of the immobilised primers. The reaction could take place in a permanently-sealed reaction chamber or chambers. During the amplification reaction, product amplicon from the solution phase lands on the solid phase oligos, modifying and extending the specific oligos. In a sealed system with low buffering, protons will be generated close to solid phase during the extension stage of each cycle of the amplification reaction, and these can be detected.

The amplification can be asymmetric, where the two primers in solution are present in different concentrations, giving rise to amplification products which are largely single stranded due to the lack of a complementary primer to extend against them. Pushing the asymmetry to give a significant excess of forward primer in the solution phase will effectively generate a "mini Run off" burst of protons in each cycle which should become detectable in the later cycles. An alternative to an asymmetry in the primers at the start of the amplification reaction, additional primer could be released or added during the amplification reaction, or at set time points. The amplification reaction will be in low buffer to allow real time detection of the pH signal.

The source nucleic acid may be a genomic polynucleotide. The source material may be eukaryotic, prokaryotic, or archaeal. One or more source materials may be provided. The source nucleic acid may represent a fragment of a genome; for example, a single chromosome, or a single genomic locus (for example, for rapid sequencing of allelic polymorphisms). In particular examples the amplification may be specific for pathogenic material within a sample. For example the amplification may select bacterial or viral nucleic acids present within a human sample. Templates may be DNA, RNA, or the cDNA copies thereof.

The biological material may be amplified in solution prior to undergoing the claimed amplification reactions. Thus the material may already have undergone a step of sequence based amplification prior to the invention being performed. Alternatively the sample may be processed to attach a universal sequence common to one or both ends of all the strands in the sample. The universal sequence can be used as a universal sequence to hybridise to a common primer, which can be non-naturally occurring.

The invention described herein can allow for four or more levels of amplification specificity. A first amplification can be carried out in solution. A second amplification can be carried out using the non-immobilised primers. A third amplification can be carried out using immobilised primers of different sequence to the second amplification primers. The presence of the amplicon can be detected using a primer specific to the amplicon generated by the immobilised primers. Thus four levels of amplification specificity can be used, ensuring that the final amplicon is only detected if the originating sequence is absolutely definitely present in the sample. Thus false positive results from the detection of closely related, but undesired sequences can be eliminated.

Immobilisation of nucleic acids to surfaces is conventional. Any method of covalent or non covalent immobilisation may be used. The immobilisation is ideally stable to multiple cycles of thermocycling to a temperature causing nucleic acid strand separation. Particular methods of immobilisation include UV induced cross-linking or the immobilisation via the formation of amide bonds or phosphorothioate bonds.

### Examples

Experimental data has been gathered for Single - Localised Anchored Primer Amplification and Detection (LAPAD) of *Serratia marcescens* (vendor: NCTC, catalogue number 27137) at a single target region and Multiple - Localised Anchored Primer Amplification and Detection (LAPAD) of *Schizosaccharomyces pombe* (vendor: ATCC, catalogue number: 26189) at multiple target regions.

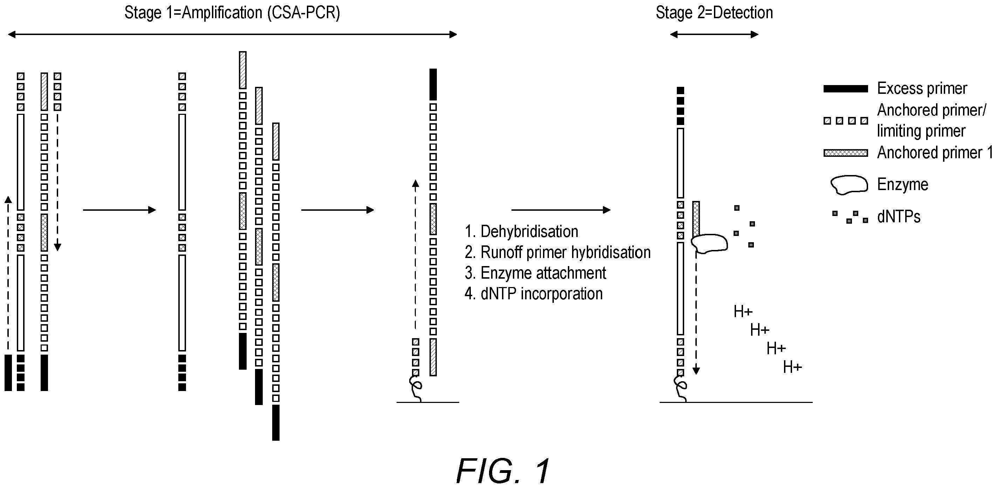

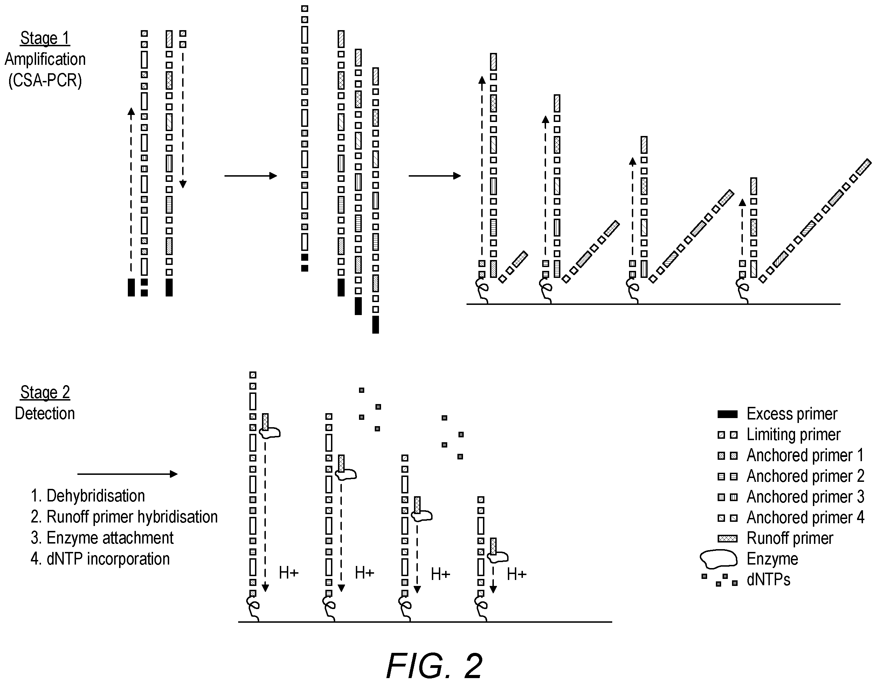

Schematic representations of both Single and Multiple - LAPAD are shown as **Figure 1** and **Figure 2****.**

### Example 1: Single Localised Anchored Primer Amplification and Detection (LAPAD)

Amplification and detection of *Serratia marcescens* at a single target region. The target region which is at the 3' end of the amplified template DNA hybridised onto the complementary anchored primer target. During the amplification process this anchored primer target was extended and in the next stage the same target region was detected during extension. Primer/oligonucleotide sequences used are shown in **Table 1.**

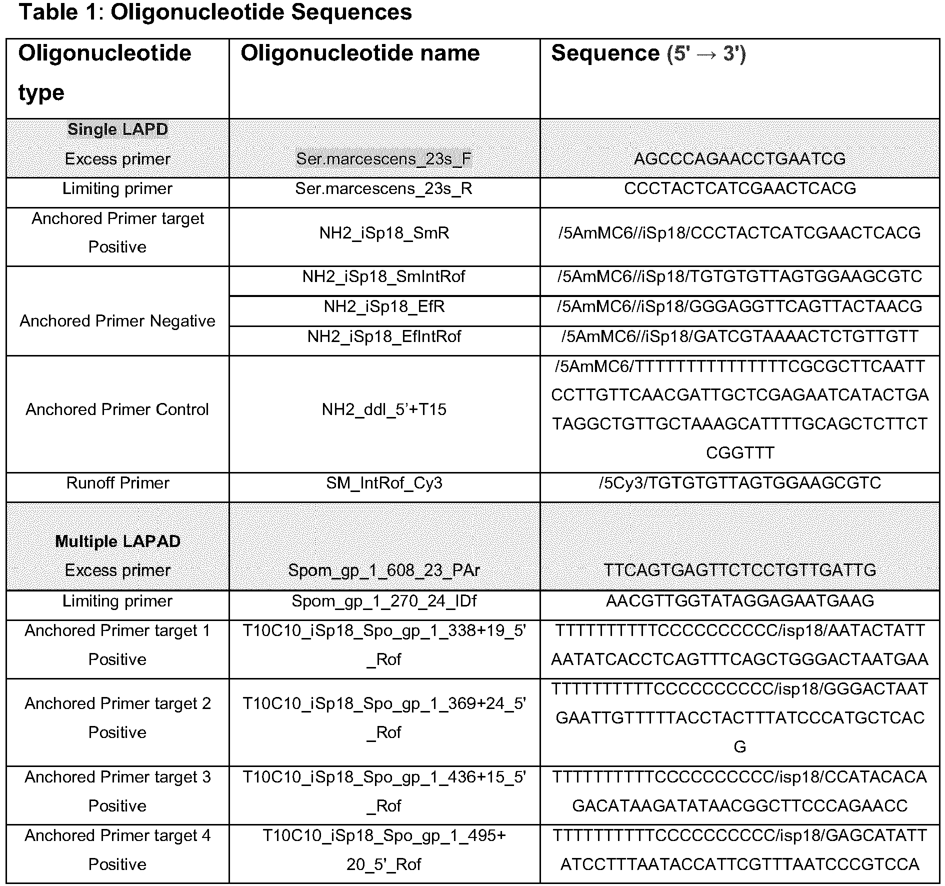

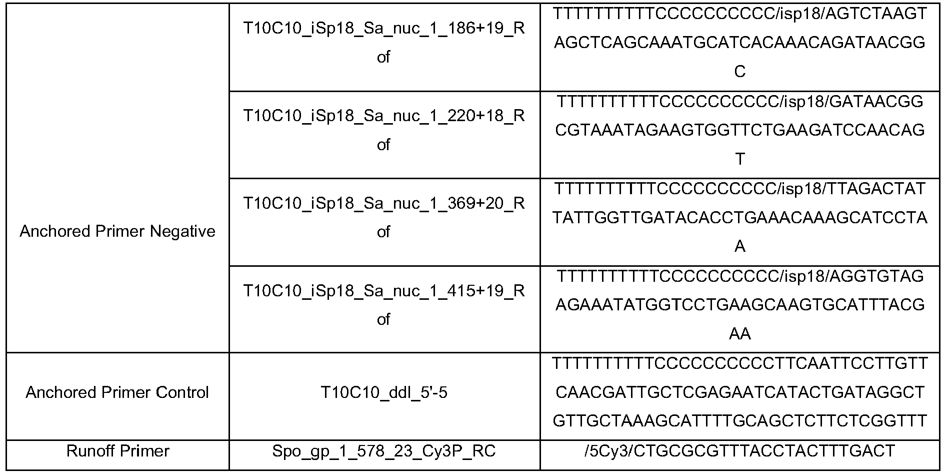

### Example 2: Multiple Localised Anchored Primer Amplification and Detection (LAPAD)

Amplification and detection of *Schizosaccharomyces pombe* at multiple target regions.

Target region 1 which is after a few nucleotide bases from the 3' end of the amplified template DNA hybridised onto the complementary anchored primer target 1. Target region 2 which after a few bases from target region 1 and towards the 5' end of the amplified template DNA hybridised onto the complementary anchored primer target 2. Target region 3 which is after a few bases from target region 2 and towards the 5' end of the amplified template DNA hybridised onto the complementary anchored primer target 3. Target region 4 which after a few bases from target region 3 and towards the 5' end of the amplified template DNA hybridised onto the complementary anchored primer target 4. During the amplification process these 4 anchored primer targets with the hybridised DNA templates were extended and then in the next stage the same target regions were detected during extension. Primer/oligonucleotide sequences used are shown in **Table 1.**

### Experimental method

The chip platform incorporated '004' Ta₂O₅ CMOS Chips consisting of ISFETs and enclosed in SU-8 wells were anchored with primers using a UV based cross-linking surface chemistry. A manifold was mounted onto the chip surface to include fluidics for the reaction to occur on the chip surface.

PCR was performed to generate DNA for detection, the PCR reagent recipe, final volume 50µl, is shown in **Table 2.** Thermocycling conditions used are shown in **Table 3.**

**Table 2: PCR Reagent Recipe**

| **Reagent** | **Vendor** | **Catalogue No.** | **[Final]** | **Units** |
|---|---|---|---|---|
| DNA free Water | Roche | 03315932001 | - | - |
| 10X TITANIUM Taq PCR Buffer | Clonetech | 639209 | 1 | X |
| Potassium chloride | Sigma Aldrich | 60142 | 34 | mM |
| Betaine | Sigma Aldrich | B0300 | 0.5 | M |
| Bovine Serum Albumin | Sigma Aldrich | B8667-5ML | 1 | mg/mL |
| Glycerol | Sigma Aldrich | G5516-100ML | 5 | % |
| Triton X-100 | Sigma Aldrich | T8787 | 0.1 | % |
| dNTPs (each) | Thermo Fisher | R0186 | 0.06 | mM |
| Excess primer | IDT | Custom | 0.5 | µM |
| Limiting primer | IDT | Custom | 0.15 | µM |
| 50x TITANIUM Taq DNA Polymerase | Clonetech | 639209 | 2.5 | X |
| Genomic DNA | See below | See below | 10,000 | Copies/µL |

**Table 3: Thermocycling Conditions**

| Temperature (°C) | Time (s) | Cycles |
|---|---|---|
| 95 | 60 | 1 |
| 95 | 5 | 40 |
| 59 | 10 | |
| 72 | 5 | |
| 95-60 | 2°C/minute | 1 |
| 60 | 120 | |
| 72 | 5 | |

Post PCR, the chips were washed with wash buffer (0.06x Saline-Sodium Citrate, 0.06% Tween20), followed by chemical dehybridisation of DNA with 20 mM NaOH for 5 minutes and then washed with wash buffer again.

Runoff primer mix (sequence shown in **Table 1)** was then added to the chip surface. 3.33 µM of fluorescently labelled (Cy3) primer was added to Annealing buffer (5 mM Magnesium Acetate, 150 mM Sodium Chloride, 20 mM Tris pH 7.5, 0.01% Tween20).

The chips with the Runoff primer mix was then heated at 95°C for 2minutes followed by 58°C for 5minutes and left for incubation at room temperature for 15minutes. Chips were then washed with Enzyme loading buffer (1x Thermopol^{®}, 0.06% Tween20).

Enzyme mix was then added onto the surface of the chip, this enzyme mix was created by adding 25units/ µL of Custom made Bst Large Fragment DNA polymerase to Enzyme loading buffer. The chips were then incubated at room temperature for 5 minutes.

Deoxyribonucleotide triphosphate (dNTP) incorporation was achieved via use of a custom-made flow system enabling dNTPs to be flown on the chip surface. 12.5 µM each of Deoxyadenosine triphosphate, Deoxythymidine triphosphate, Deoxyguanosine triphosphate and Deoxycytidine triphosphate was added to non-carbonated RMD buffer (10 mM Magnesium Chloride, 25 mM Sodium Chloride, 0.025% Tergitol). The pH of the dNTP mix was adjusted to pH 8 and maintained by keeping the fluid under nitrogen gas.

### ISFET sensor detection

The raw ISFET sensor voltage output data and Anchored primer spotting map was processed together by a custom data analysis pipeline (Galaxy Runoff pipeline v1.3.15). Voltage signal peak heights from ISFETs were detected and Anchored primer ISFET voltage signal peak heights were subtracted from that of the adjacent upstream blank ISFET. This is done to remove system noise including noise from the fluidic flow which may mask detection. This delta ISFET sensor voltage output from all the specific Anchored primers sensors were averaged to determine signal detection.

### E-gel electrophoresis analysis

Readymade E-gels (48 well 2% agarose gels stained with Ethidium Bromide) were used for qualitative analysis of solution phase PCR. Samples were collected post PCR from the chip surface and 1 uL of the sample was adding to 1x E-gel loading dye before loading into the E-gel wells. A ladder was also loaded for size comparison of the amplicon. The gel was run for 10minutes on the E-gel base which provides an electric field for the DNA amplicon to migrate according to its size.

### Sensospot fluorescence imaging

Post run, chips were scanned in the Sensospot fluorescence scanner under a green light to illuminate the Cy3 runoff primer which would have hybridised onto the on-chip generated amplicon. Based on the fluorescence intensity, the on-chip amplification can be determined qualitatively.

### Results

Detection for both assays was established quantitively by the delta ISFET sensor voltage output. The Solution phase PCR amplification was qualitatively assessed by E-gel analysis and Chip surface amplification by Sensospot fluorescent imaging.

### Example 1: Single Localised Anchored Primer Amplification and Detection (LAPAD)

Target: *Serratia marcescens*
Chip ID: NM-248-0214

Anchored primer sequences for Example 1 can be seen in **Table 4.**

**Table 4: Anchored Primer Sequences for Example 1**

| | |
|---|---|
| Anchored Primer target Positive | NH2_iSp18_SmR |
| Anchored Primer Negative | NH2_iSp18_SmIntRof |
| | NH2_iSp18_EfR |
| | NH2_iSp18_EfIntRof |
| Anchored Primer Control | NH2_ddl_5'+T15 |

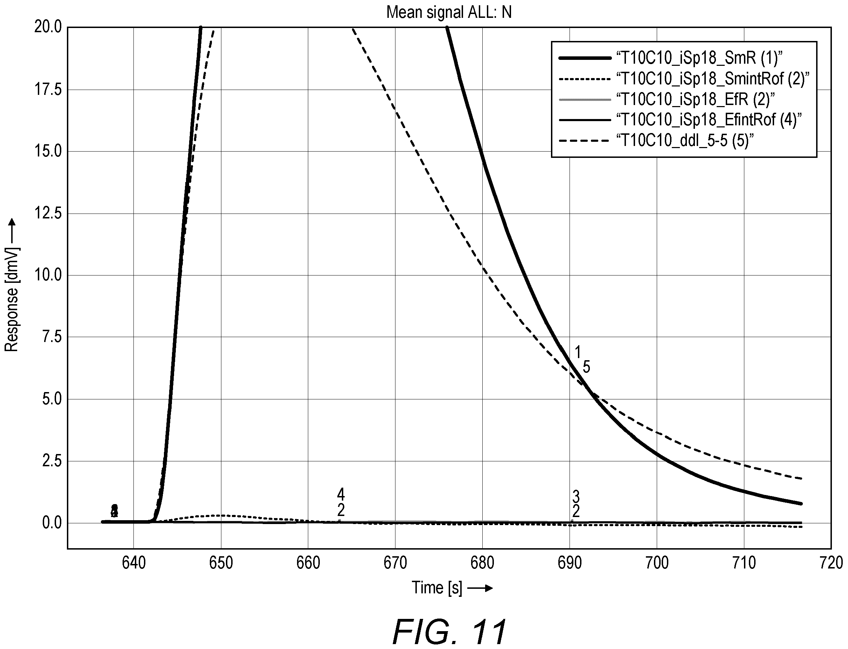

Average delta signal from ISFETs can be seen in **Figure 11****.**

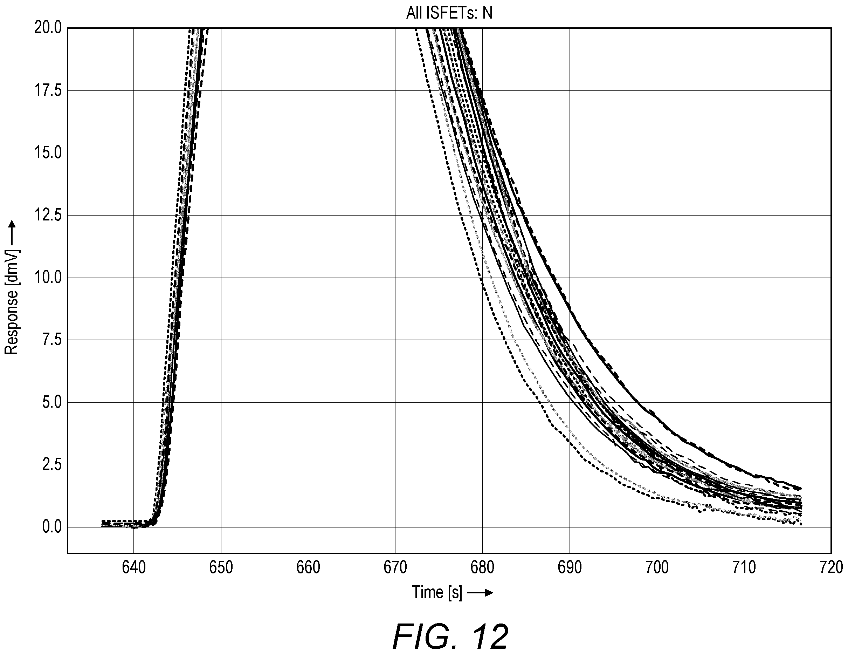

Delta signals from all ISFETs for Anchored Primer target Positive can be seen in **Figure 12****.**

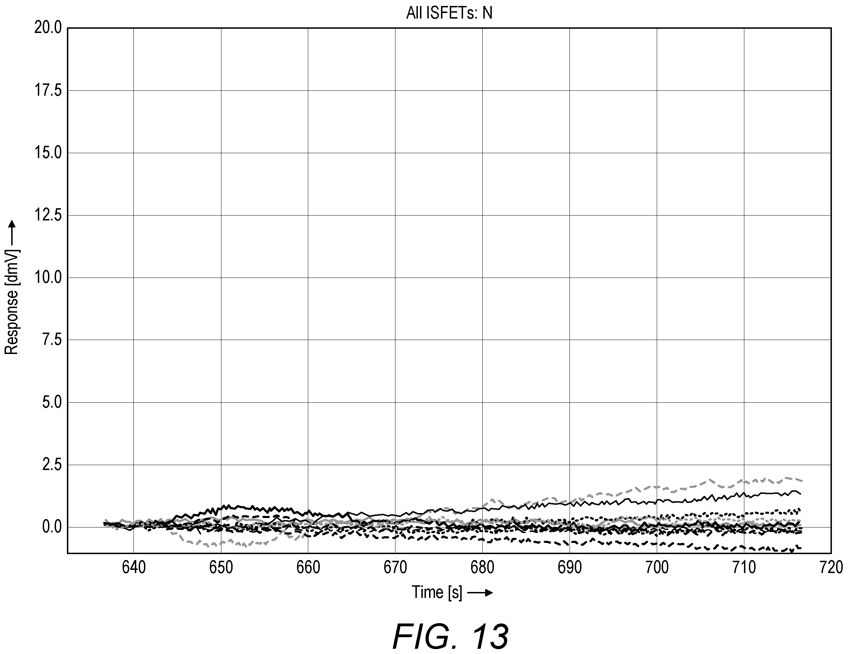

Delta signals from all ISFETs for Anchored Primer target Negative can be seen in **Figure 13**

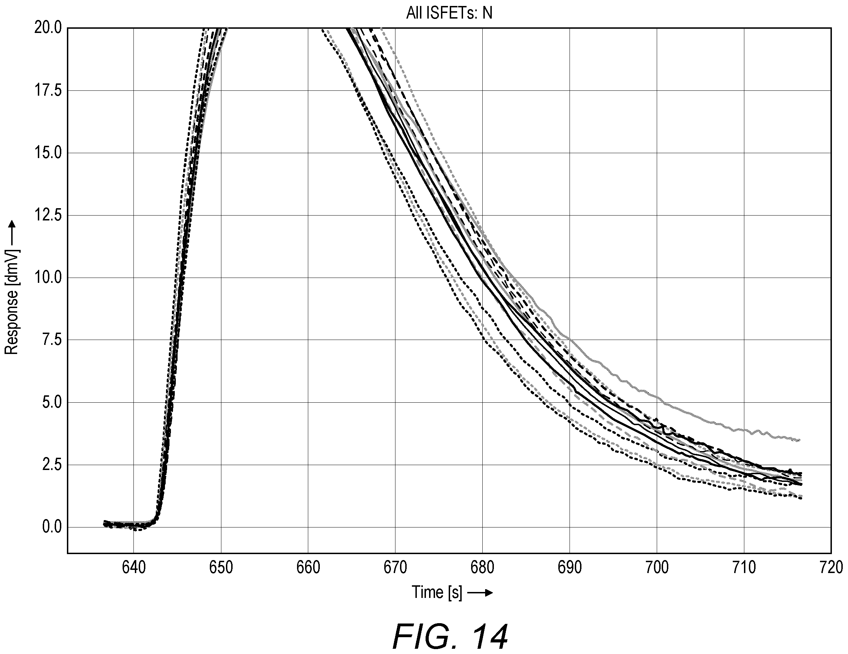

Delta signals from all ISFETs for Anchored Primer control can be seen in **Figure 14****.**

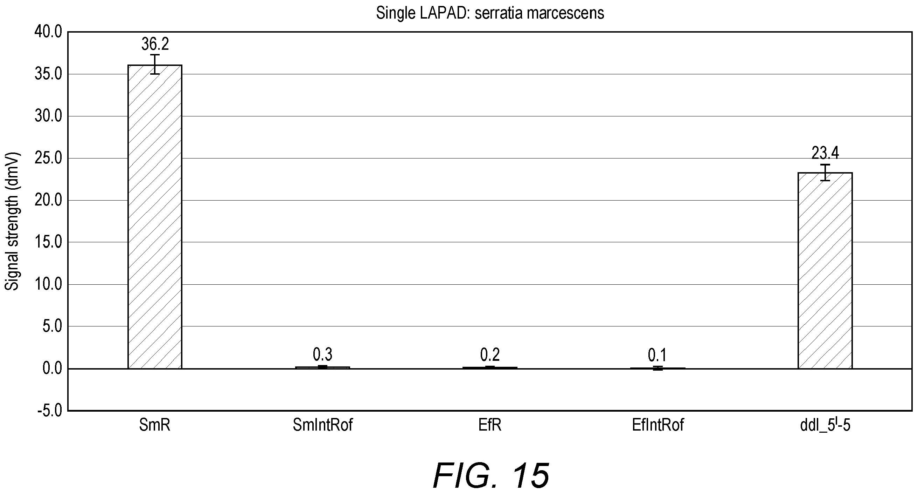

Average ISFET signal results for Positive, Negative and Control primers can be seen in **Figure 15****.** An average 36.2 dmV ISFET signal was detected for the Positive Anchored primer NH2_iSp18_SmR which was even greater than the 23.4 dmV average ISFET signal for the Control Anchored Primer NH2_ddl_5'+T15.

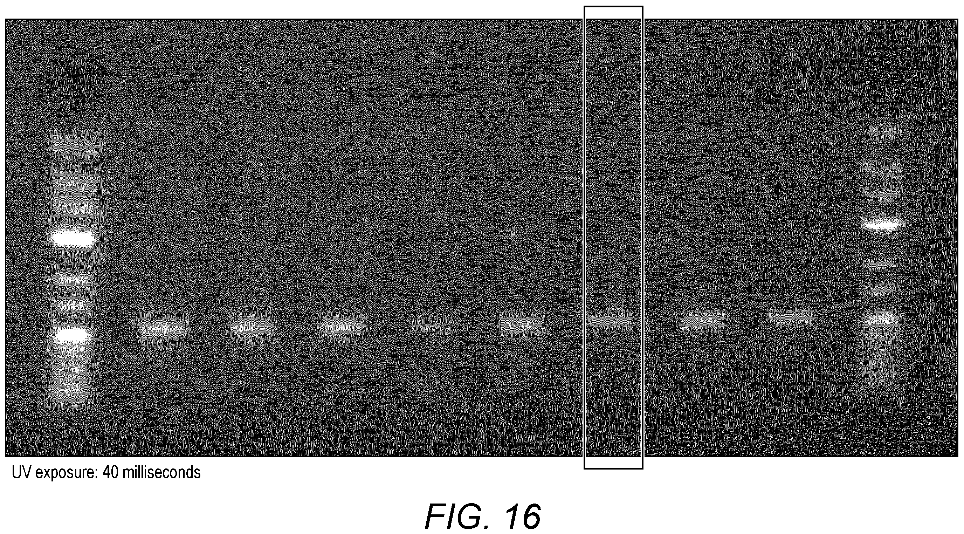

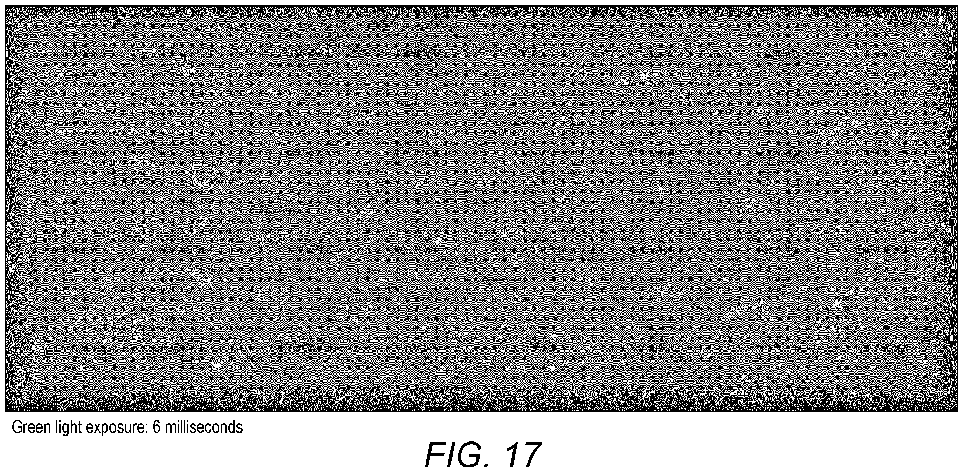

Solution phase amplicon was generated post PCR for *Serratia marcescens* as seen in the gel electrophoresis image **(****Figure 16****).** Along with the Control Anchored primer, the On-chip amplicon generated can also be seen for the Positive Anchored primer NH2-iSp18_SmR on the sensospot fluorescent image **(****Figure 17****).**

Amplification and detection of *Serratia marcescens* at a single target region was demonstrated. A significant delta ISFET voltage signal was detected which was even larger than the Control Anchored Primer.

### Example 2: Multiple Localised Anchored Primer Amplification and Detection (LAPAD)

Target: *Schizosaccharomyces pombe*
Chip ID: NM-248-0172

Anchored primer sequences for Example 2 can be seen in **Table 5.**

**Table 5: Anchored primer sequences for Example 2.**

| **Anchored Primer target 1 Positive** | **T10C10_iSp18_Spo_gp_1_338+19_5'_Rof** |
|---|---|
| Anchored Primer target 2 Positive | T10C10_iSp18_Spo_gp_1_369+24_5'_Rof |
| Anchored Primer target 3 Positive | T10C10_iSp18_Spo_gp_1_436+15_5'_Rof |
| Anchored Primer target 4 Positive | T10C10_iSp18_Spo_gp_1_495+20_5'_Rof |
| Anchored Primer Negative | T10C10_iSp18_Sa_nuc_1_186+19_Rof |
| | T10C10_iSp18_Sa_nuc_1_220+18_Rof |
| | T10C10_iSp18_Sa_nuc_1_369+20_Rof |
| | T10C10_iSp18_Sa_nuc_1_415+19_Rof |
| Anchored Primer Control | T10C10_ddl_5'-5 |

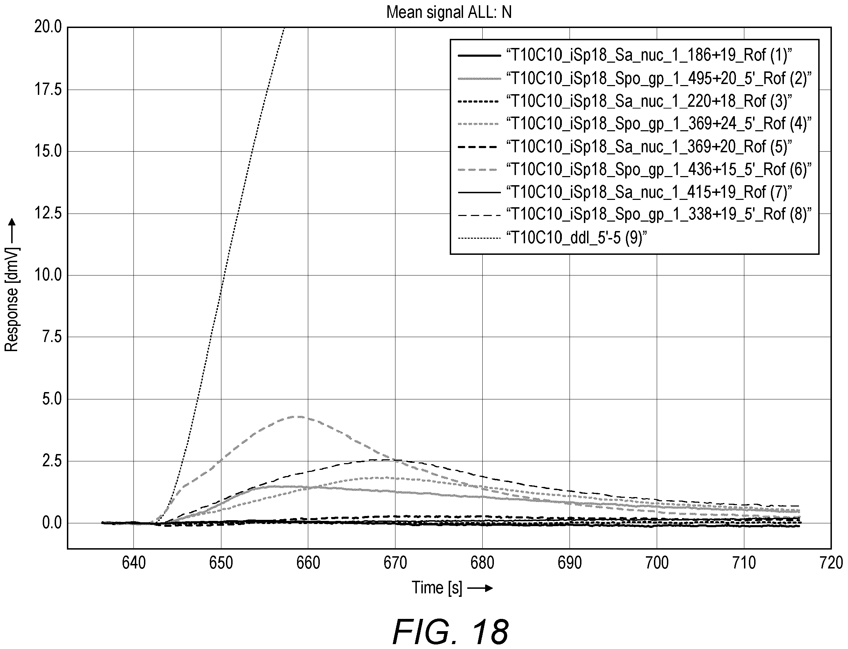

Average delta signal from ISFETs for Example 2, can be seen in **Figure 18****.**

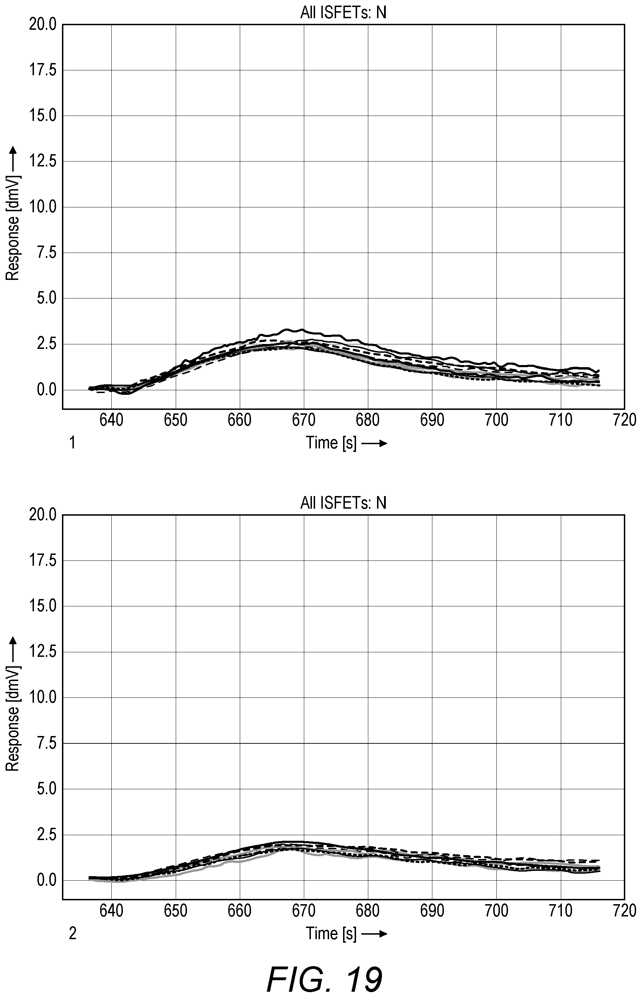

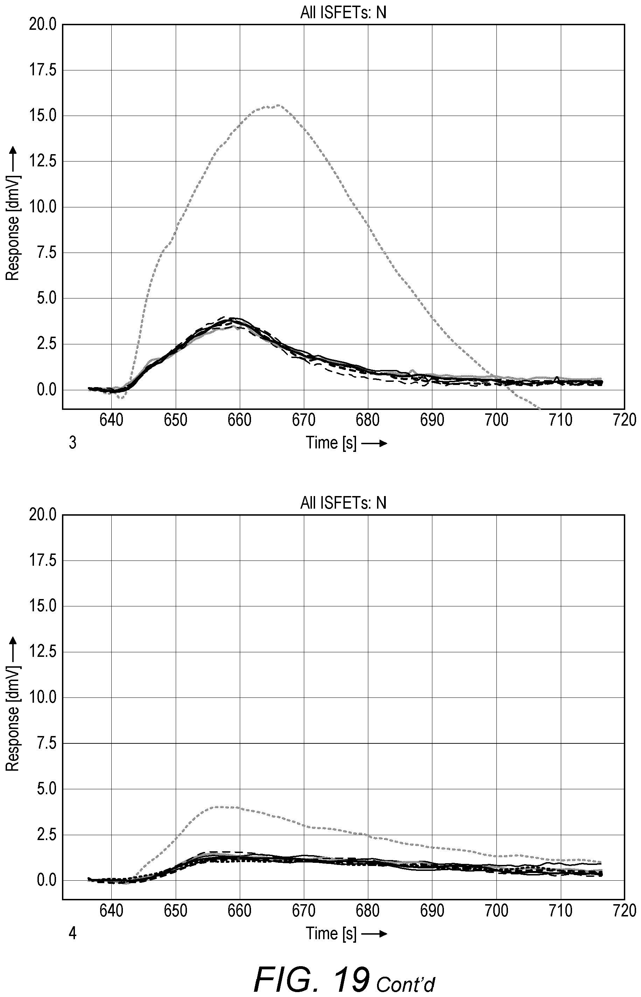

Delta signals from all ISFETs for Anchored Primer targets 1 to 4 Positive can be seen in **Figure 19****.**

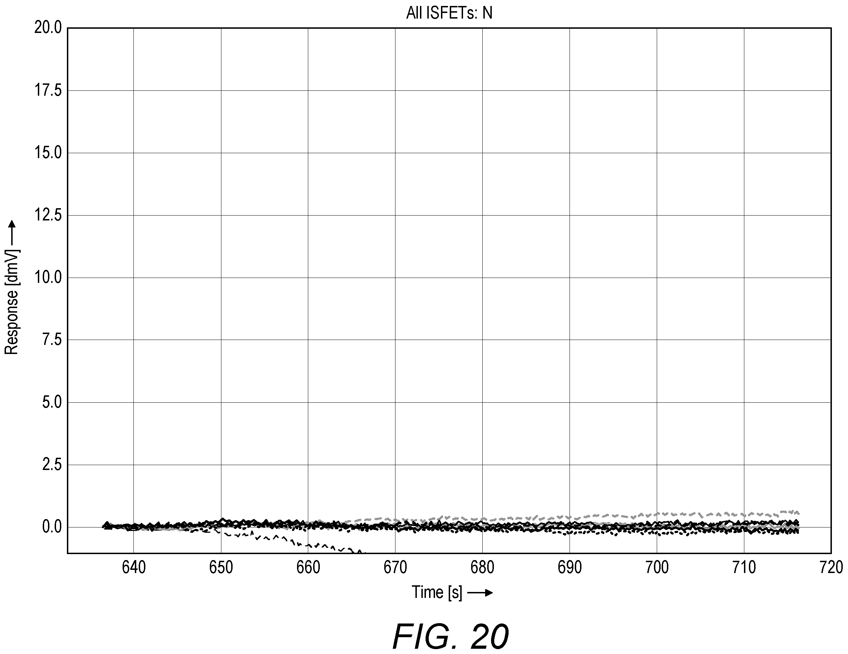

Delta signals from all ISFETs for Anchored Primer Negative can be seen in **Figure 20****.**

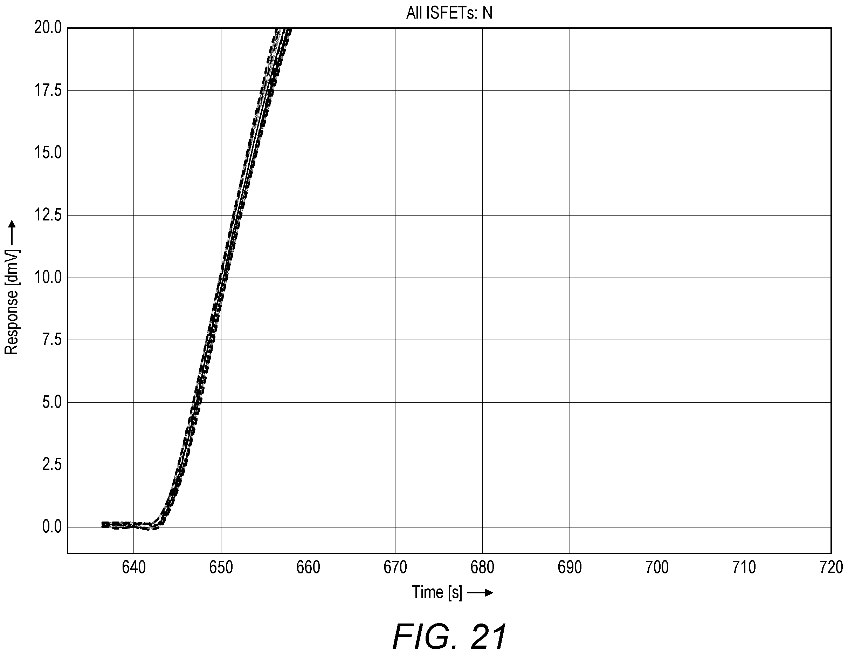

Delta signals from all ISFETs for Anchored Primer Control can be seen in **Figure 21****.**

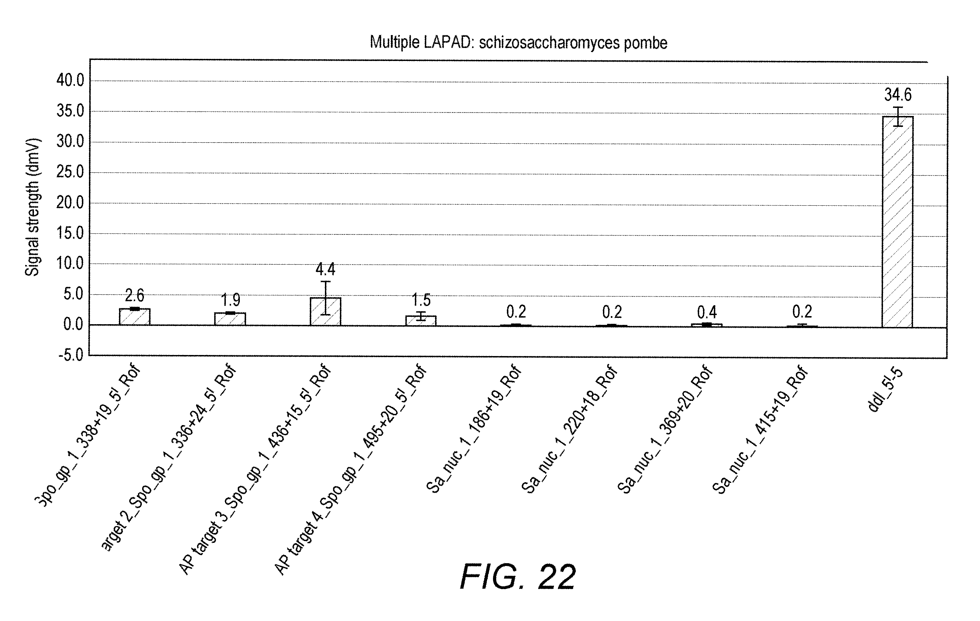

Average ISFET signal results for Positive, Negative and Control primers can be seen in **Figure 22****.** 1.5 to 4.4 dmV ISFET signal was detected for the four Positive Anchored primers. This is much lower than previous signal intensities and the Control Anchored Primer average ISFET signal which was 34.6 dmV.

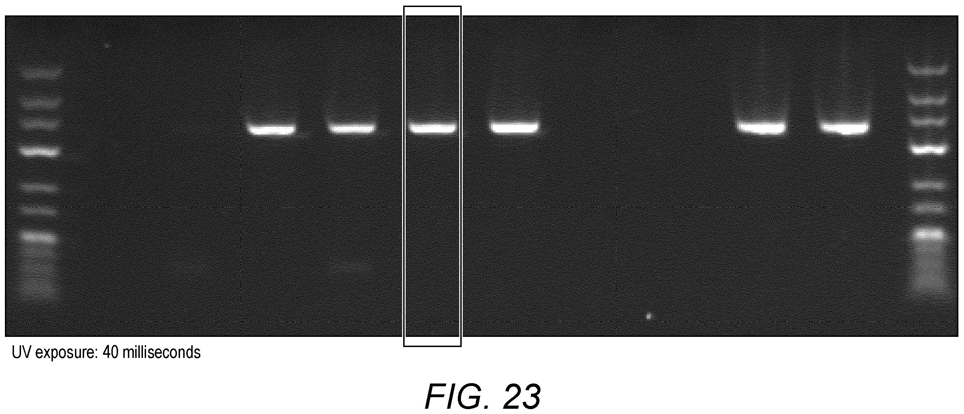

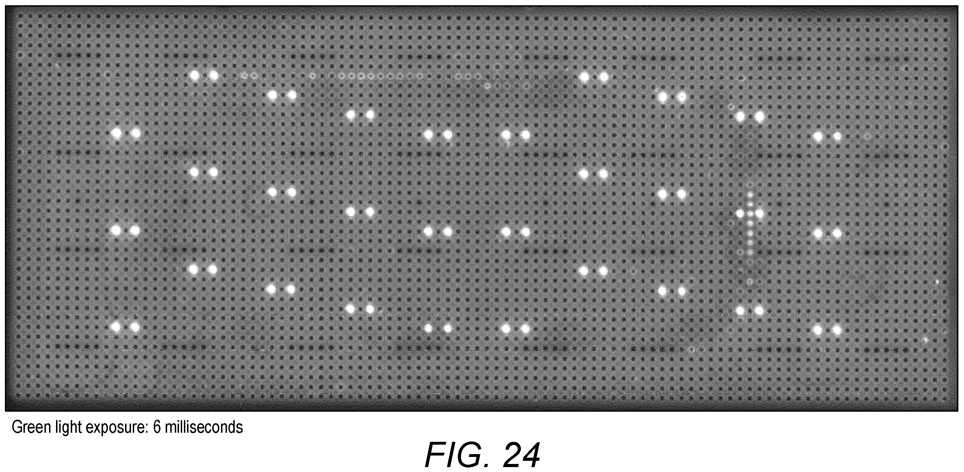

Solution phase amplicon was generated post PCR for *Schizosaccharomyces pombe* as seen in the gel electrophoresis image **(****Figure 23****).** On-chip amplicon generated cannot be seen for the Positive Anchored primers on the sensospot fluorescent image **(****Figure 24****).** The Control Anchored primer however can be seen.

Amplification and detection of *Schizosaccharomyces pombe* at multiple target regions was demonstrated.

## Claims

1. A method for the amplification and analysis of nucleic acid sequences, the method comprising:
a. taking a system having a liquid volume containing a nucleic acid sample, two or more non-immobilised amplification primers and reagents for nucleic acid amplification, and one or more immobilised amplification primers; wherein the non-immobilised amplification primers are in excess of the immobilised amplification primers;
b. performing an asymmetric nucleic acid amplification reaction using the non-immobilised amplification primers to produce a first non-immobilised nucleic acid amplification product;
c. further amplifying the first non-immobilised nucleic acid amplification product using the one or more immobilised amplification primers to produce an immobilised second nucleic acid amplification product;
d. interrogating a localised signal generated at the immobilised second nucleic acid amplification product location in real time and
e. detecting the presence, absence or sequence of the second nucleic acid amplification product.

2. The method according to claim 1, wherein the ratio of non-immobilised amplification primers to immobilised amplification primers is 3:1.

3. The method according to any of the preceding claims, wherein additional primer is released or added during the amplification reaction, or at set time points.

4. The method according to claim 1 wherein the non-immobilised amplification primers in the liquid volume are released from the solid phase prior to amplification.

5. The method according to any of the preceding claims, wherein the amplified products are detected optically.

6. The method according to any one preceding claim wherein the detection of the amplified products involves fluorescent reporter probes and subsequent measurement of fluorescence.

7. The method according to any of the preceding claims, wherein the immobilised amplification primers amplify an internal section of the first amplification product such that the second amplification product is shorter than the first amplification product.

8. The method according to any one preceding claim wherein the first liquid volume is exposed to two or more immobilised amplification primers where each primer amplifies a different sequence.

9. The method according to claim 8, wherein the two or more immobilised amplification primers differ by a single base, thereby detecting single base variants in the first nucleic amplification products.

10. The method according to any one of the preceding claims wherein the method is carried out on an array type optical imaging system comprising a surface with which the liquid volume is contacted.

## Patentansprüche

1. Verfahren zur Amplifikation und Analyse von Nukleinsäuresequenzen, wobei das Verfahren Folgendes umfasst:
a. Verwenden eines Systems mit einem Flüssigkeitsvolumen, das eine Nukleinsäureprobe, zwei oder mehr nicht immobilisierte Amplifikationsprimer und Reagenzien zur Nukleinsäureamplifikation und einen oder mehrere immobilisierte Amplifikationsprimer enthält; wobei die nicht immobilisierten Amplifikationsprimer die immobilisierten Amplifikationsprimer übersteigen;
b. Durchführen einer asymmetrischen Nukleinsäureamplifikationsreaktion unter Verwendung der nicht immobilisierten Amplifikationsprimer, um ein erstes nicht immobilisiertes Nukleinsäureamplifikationsprodukt zu erzeugen;
c. weiteres Amplifizieren des ersten nicht immobilisierten Nukleinsäureamplifikationsprodukts unter Verwendung des einen oder der mehreren immobilisierten Amplifikationsprimer, um ein immobilisiertes zweites Nukleinsäureamplifikationsprodukt zu erzeugen;
d. Abfragen eines lokalisierten Signals in Echtzeit, das an der Stelle des immobilisierten zweiten Nukleinsäureamplifikationsprodukts generiert wird, und
e. Nachweisen der Anwesenheit, Abwesenheit oder Sequenz des zweiten Nukleinsäureamplifikationsprodukts.

2. Verfahren nach Anspruch 1, wobei das Verhältnis von nicht immobilisierten Amplifikationsprimem zu immobilisierten Amplifikationsprimern 3:1 beträgt.

3. Verfahren nach einem der vorhergehenden Ansprüche, wobei während der Amplifikationsreaktion oder zu festgelegten Zeitpunkten zusätzlicher Primer freigesetzt oder hinzugefügt wird.

4. Verfahren nach Anspruch 1, wobei die nicht immobilisierten Amplifikationsprimer in dem Flüssigkeitsvolumen vor der Amplifikation aus der festen Phase freigesetzt werden.

5. Verfahren nach einem der vorhergehenden Ansprüche, wobei die amplifizierten Produkte optisch nachgewiesen werden.

6. Verfahren nach einem vorhergehenden Anspruch, wobei der Nachweis der amplifizierten Produkte fluoreszierende Reportersonden und eine anschließende Messung der Fluoreszenz einbezieht.

7. Verfahren nach einem der vorhergehenden Ansprüche, wobei die immobilisierten Amplifikationsprimer einen inneren Abschnitt des ersten Amplifikationsprodukts amplifizieren, so dass das zweite Amplifikationsprodukt kürzer als das erste Amplifikationsprodukt ist.

8. Verfahren nach einem vorhergehenden Anspruch, wobei das erste Flüssigkeitsvolumen zwei oder mehreren immobilisierten Amplifikationsprimem ausgesetzt wird, wobei jeder Primer eine andere Sequenz amplifiziert.

9. Verfahren nach Anspruch 8, wobei sich die zwei oder mehreren immobilisierten Amplifikationsprimer um eine einzige Base unterscheiden, wodurch Einzelbasenvarianten in den ersten Nukleinsäureamplifikationsprodukten nachgewiesen werden.

10. Verfahren nach einem der vorhergehenden Ansprüche, wobei das Verfahren auf einem optischen Abbildungssystem vom Array-Typ ausgeführt wird, das eine Oberfläche umfasst, mit der das Flüssigkeitsvolumen in Kontakt kommt.

## Revendications

1. Procédé permettant l'amplification et l'analyse de séquences d'acide nucléique, le procédé comprenant :
a. la prise d'un système comportant un volume de liquide contenant un échantillon d'acide nucléique, au moins deux amorces d'amplification non immobilisées et des réactifs pour amplification de l'acide nucléique, et une ou plusieurs amorces d'amplification immobilisées ; dans lequel les amorces d'amplification non immobilisées sont supérieures aux amorces d'amplification immobilisées ;
b. l'exécution d'une réaction d'amplification de l'acide nucléique asymétrique en utilisant les amorces d'amplification non immobilisées pour produire un premier produit d'amplification de l'acide nucléique non immobilisé ;
c. une nouvelle amplification du premier produit d'amplification de l'acide nucléique non immobilisé en utilisant le ou les amorces d'amplification immobilisées pour produire un second produit d'amplification de l'acide nucléique immobilisé ;
d. l'interrogation d'un signal localisé généré à l'emplacement du second produit d'amplification de l'acide nucléique immobilisé en temps réel et
c. la détection de la présence, de l'absence ou de la séquence du second produit d'amplification de l'acide nucléique.

2. Procédé selon la revendication 1, dans lequel le rapport des amorces d'amplification non immobilisées aux amorces d'amplification immobilisées est de 3:1.

3. Procédé selon l'une quelconque des revendications précédentes, dans lequel une amorce supplémentaire est libérée ou ajoutée pendant la réaction d'amplification ou à des points fixés dans le temps.

4. Procédé selon la revendication 1, dans lequel les amorces d'amplification non immobilisées dans le volume de liquide sont libérées de la phase solide avant l'amplification.

5. Procédé selon l'une quelconque des revendications précédentes, dans lequel les produits amplifiés sont détectés optiquement.

6. Procédé selon l'une quelconque des revendications précédentes, dans lequel la détection des produits amplifiés fait usage de sondes rapporteuses fluorescentes et de la mesure consécutive de la fluorescence.

7. Procédé selon l'une quelconque des revendications précédentes, dans lequel les amorces d'amplification immobilisées amplifient une section interne du premier produit d'amplification de telle sorte que le second produit d'amplification soit plus court que le premier produit d'amplification.

8. Procédé selon l'une quelconque des revendications précédentes, dans lequel le premier volume de liquide est exposé à au moins deux amorces d'amplification immobilisées où chaque amorce amplifie une séquence différente.

9. Procédé selon la revendication 8, dans lequel les deux amorces d'amplification immobilisées ou plus diffèrent par une base simple, en détectant ainsi les variantes de base simple dans les premiers produits d'amplification nucléique.

10. Procédé selon l'une quelconque des revendications précédentes, le procédé étant exécuté sur un système d'imagerie optique de type tableau comprenant une surface avec laquelle le volume de liquide est mis en contact.
